(19) **Europäisches Patentamt European Patent Office Office européen des brevets**

(11) **EP 4 558 191 B1**

(12) **FASCICULE DE BREVET EUROPEEN**

(45) Date de publication et mention
de la délivrance du brevet:
**27.05.2026 Bulletin 2026/22**

(21) Numéro de dépôt: **23742314.0**

(22) Date de dépôt: **17.07.2023**

(51) Classification Internationale des Brevets (IPC):
*A61M 1/36* (2006.01)     *A61M 60/109* (2021.01)
*A61M 60/117* (2021.01)     *A61M 60/268* (2021.01)
*A61M 60/443* (2021.01)     *A61M 60/515* (2021.01)
*A61M 60/577* (2021.01)

(52) Classification Coopérative des Brevets (CPC):
**A61M 60/117; A61M 1/3666; A61M 60/109;
A61M 60/268; A61M 60/443; A61M 60/515;
A61M 60/577**

(86) Numéro de dépôt international:
**PCT/EP2023/069813**

(87) Numéro de publication internationale:
**WO 2024/017840 (25.01.2024 Gazette 2024/04)**

(54) **DISPOSITIF D'ASSISTANCE OU DE SUPPLÉANCE DU COEUR**

HERZUNTERSTÜTZUNGS- ODER -SICHERUNGSVORRICHTUNG

HEART ASSISTANCE OR BACK-UP DEVICE

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **18.07.2022 FR 2207327**

(43) Date de publication de la demande:
**28.05.2025 Bulletin 2025/22**

(73) Titulaire: **Bypa Medical Solutions
21000 Dijon (FR)**

(72) Inventeur: **DE BROSSES, Bertrand
21000 Dijon (FR)**

(74) Mandataire: **Roman, Alexis
SPE Roman-André
35, rue Paradis
B.P. 30064
13484 Marseille Cedex 20 (FR)**

(56) Documents cités:
DE-A1- 10 020 546     US-A1- 2003 032 853
US-A1- 2010 268 334

EP 4 558 191 B1

**Description**

**Domaine technique.**

La présente invention a pour objet un dispositif d'assistance ou de suppléance du cœur.
Le terme assistance est utilisée lorsqu'une partie du sang arrivant au cœur est prélevée tandis que le terme de suppléance est utilisé lorsque la totalité, ou quasi-totalité (> 90%), du sang arrivant au cœur est prélevée par le dispositif de suppléance du cœur.
Elle concerne le domaine technique des dispositifs utilisés par une assistance/suppléance circulatoire extra-corporelle qui maintiennent une hémodynamique compatible avec la vie, dans le cadre d'une défaillance du muscle cardiaque, ou plus généralement cardio-pulmonaire, menaçant le pronostic vital du patient, suite à une insuffisance cardiaque aiguë ou chronique (insuffisance coronarienne et/ou une maladie cardio-vasculaire, ou autre pathologie associée).

**État de la technique.**

Le cœur a pour fonction de distribuer le sang dans l'organisme pour transporter l'oxygène et les nutriments nécessaires au fonctionnement des différents organes et de transporter les déchets du métabolisme vers son organe d'élimination du $CO^2$ les poumons, urée et créatinine vers les reins. Il est divisé en deux parties, le cœur droit qui reçoit par les veines caves le sang veineux (appauvri en oxygène) et qui l'envoie par le ventricule droit vers l'artère pulmonaire vers le système respiratoire (ou il est rechargé en oxygène et déchargé en $CO^2$), et le cœur gauche qui reçoit par les veines pulmonaires le sang oxygéné pour l'éjecter par l'aorte vers les différents organes. Les contractions du muscle cardiaque (pompe) permettent de générer un débit sanguin pulsé dans l'organisme. Elles permettent un asservissement continu du débit sanguin aux besoins physiologiques de l'organisme et de chaque organe.
En présence d'une défaillance du muscle cardiaque (en cas d'infarctus du myocarde par exemple), il est nécessaire de suppléer temporairement à la fonction de pompage dudit muscle cardiaque par un système mécanique d'assistance circulatoire, l'objectif étant de garantir la bonne circulation sanguine pendant que le coeur récupère.
Parmi les systèmes d'assistance circulatoire actuellement utilisés, on connaît des dispositifs de circulation implantables, par exemple décrit dans le document brevet US2003/032853, mais dont la mise en place est particulièrement complexe et risquée. On connait également les dispositifs de circulation extracorporelle (en abrégé CEC) permettant de court-circuiter le cœur défaillant en utilisant une pompe asservie placée à l'extérieur du corps, qui reçoit le sang au niveau des veines caves et l'injecte au niveau de l'aorte (thoracique), pour générer mécaniquement un débit sanguin continu compatible avec les besoins vitaux de l'organisme, puis procéder à l'opération. Le principal objectif habituellement recherché lors d'une assistance circulatoire est de réguler le débit pompé en complète adéquation avec les besoins physiologiques du patient, ces derniers variant continuellement.
En tant que source d'énergie, une console ou une unité centrale comporte un modèle mathématique conçu à partir de lois physiques régissant le déplacement d'un fluide dans un circuit fermé. Ce circuit est généralement composé d'une pompe, d'un échangeur thermique, d'un débitmètre, d'un analyseur des gaz et d'électrolytes sanguins, d'un capteur de pression ainsi que d'équipements biocompatibles comme les tubulures, les canules artérielle et veineuse, le réservoir veineux, l'oxygénateur, le filtre artériel. Habituellement une pompe centrifuge ou péristaltique est utilisée comme pompe de tête artérielle et quatre autres pompes péristaltiques sont utilisées pour l'aspiration de la cardiotomie, la circulation des chambres cardiaques, l'administration de la cardioplégie et une pompe de secours. Si une défaillance persiste, l'ECMO (« Extracorporeal Membrane Oxygenation ») ou l'ECLS (« Extracorporeal Life Support ») est indiqué. Ces systèmes sont plus simples qu'une CEC standard et sont transportables, le temps d'utilisation étant de plusieurs jours contrairement à la CEC classique. En effet, à la différence de la CEC classique, l'ECMO comme l'ECLS sont maintenus jusqu'à la récupération cardiopulmonaire du patient ou comme relais avant une transplantation.
Bien que ces systèmes aient démontré leur efficacité dans leurs fonctions d'assistance circulatoire conventionnelle, ils ne sont pas parfaitement satisfaisants dans la mesure où l'asservissement du débit sanguin généré n'est pas optimal au regard de la physiologie du patient. Le taux de survie actuel pour certaines pathologies adressées n'excède pas 30%. Un cœur fatigué ou défaillant se fatiguera encore plus si la pression aortique est forte pendant la systole : les valves aortiques s'ouvrent mal, la post charge augmente et la pression télé-diastolique du ventricule gauche aussi avec une absence d'éjection. On constate également une fuite de la valve mitrale susceptible d'occasionner un œdème pulmonaire irréversible. Il s'agit d'une des principales limites des systèmes de l'art antérieur.
Le document brevet WO2006/016047 (BERTHIER) décrit un dispositif d'assistance circulatoire extracorporelle pour le cœur pourvu d'un moteur linéaire configuré pour déplacer en translation un piston dans un réservoir de manière à provoquer un écoulement de sang caractérisé par une succession de phases d'aspiration et de phases d'éjection. Le moteur linéaire est asservi pour piloter le piston en déplacement, vitesse et accélération en fonction des besoins physiologiques du patient. Toutefois, il a été montré que l'adéquation entre l'écoulement pulsatile et les besoins physiologiques du patient n'est pas optimale et ne permet pas d'obtenir la performance recherchée.

Un dispositif d'assistance circulatoire implantable a également été décrit dans le document US 2003/032853 A1 (KORAKIANITIS THEODOSIOS). Ce dispositif comprend un actionneur linéaire destiné à provoquer un écoulement sanguin pulsatile en déplaçant un organe dans une chambre. Il est conçu pour soutenir l'activité du cœur en éjectant du sang vers la circulation systémique, sur la base d'une loi de contrôle prenant en compte des paramètres hémodynamiques du patient (tels que des signaux ECG), sans toutefois mettre en œuvre une succession de phases distinctes d'aspiration et d'éjection du fluide, ni permettre un asservissement de la pression d'éjection selon les besoins physiologiques évalués en temps réel. En outre, le dispositif est implantable, ce qui rend sa mise en place invasive et plus complexe.

La présente invention vise à pallier tout ou partie des inconvénients précités. En particulier, un objectif de l'invention est de proposer un dispositif d'assistance ou de suppléance dont le fonctionnement permet de maximiser les avantages de l'assistance ou de la suppléance.

Un autre objectif de l'invention est d'optimiser la synchronisation en temps réel des phases d'éjection au regard de la physiologie du patient pour conserver une hémodynamique compatible avec la vie dudit patient.

Encore un autre objectif de l'invention est de proposer un dispositif d'assistance ou de suppléance dont l'asservissement est simple à mettre en œuvre et particulièrement fiable et robuste aux changements des paramètres physiologiques du patient.

L'invention a également pour objectif de permettre de meilleures performances et une précision accrue des cycles de pompage.

## Présentation de l'invention.

La solution proposée par l'invention est un dispositif d'assistance ou de suppléance circulatoire extracorporelle temporaire du coeur d'un patient, comprenant :

- un actionneur linéaire configuré pour déplacer en translation un organe dans une chambre de manière à provoquer un écoulement de fluide pulsatif apte à soutenir l'activité du cœur dudit patient, lequel écoulement est caractérisé par une succession de phases d'aspiration et de phases d'éjection du fluide,
- une unité de commande configurée pour piloter l'actionneur et contrôler le déplacement de l'organe, lequel pilotage est réalisé en prenant en compte des données d'entrée provenant de mesures de paramètres hémodynamiques du patient, durant chaque phase d'éjection ladite unité déplaçant l'organe d'une distance D.

L'unité de commande est configurée pour :

- séquencer le déplacement D en un nombre entier N de cycles $C_i$, avec N>1, chaque cycle ayant la même durée $\Delta t$,

- sur chaque cycle $C_i$, calculer une longueur de déplacement $L_i$ de l'organe telle que $D = \sum_{i=1}^{N} Li$, laquelle longueur $L_i$ est calculée en fonction des données d'entrée provenant :

  ∘ d'un ou plusieurs paramètres hémodynamiques du patient mesurés pendant le cycle $C_i$, ou pendant un ou plusieurs cycles précédents ledit cycle $C_i$ et proches dudit cycle $C_i$,
  ∘ de la valeur d'une pression de consigne $\Pi_i$ basée sur la loi de Franck-Starling,

- sur chaque cycle $C_i$, transmettre une instruction à l'actionneur pour déplacer l'organe de la longueur $L_i$ calculée.

Sur chaque cycle $C_i$, le pilotage de la longueur $L_i$ permet d'ajuster de manière optimale la vitesse de déplacement de l'organe selon les paramètres hémodynamiques du patient et, de fait, la pression du fluide éjecté durant ledit cycle aux besoins physiologiques dudit patient. Il en résulte que durant chaque phase d'éjection, la pression du fluide éjecté peut être très précisément en adéquation avec la courbe de Franck-Starling en réponse aux changements de paramètres hémodynamiques du patient à chaque cycle $C_i$. Selon la loi de Franck-Starling, plus l'étirement des fibres musculaires cardiaques est important (produit par la précharge), plus le volume d'éjection systolique est élevé. Autrement dit, le volume de sang éjecté par battement sera proportionnel au volume de sang contenu dans le ventricule en fin de diastole.

Selon l'invention, la pression d'éjection n'est pas adaptée à une valeur de pression cible constante, mais aux besoins réels du patient pour retrouver ce mécanisme de Frank-Starling. Le dispositif selon l'invention permet ainsi de maximiser les avantages de l'assistance ou de la suppléance, tout en restant particulièrement fiable et robuste aux changements des besoins physiologiques du patient. La demanderesse a constaté de manière surprenante que le taux de survie après 15 jours d'assistance ou de suppléance avec le dispositif selon l'invention, pouvait monter à 80%.

En outre, en séquençant le déplacement de l'organe durant la phase d'éjection et en adaptant sa longueur de déplacement sur chaque cycle de la séquence, les cycles de pompage ont une précision accrue dans la mesure. En effet, la valeur de la pression d'éjection sur la courbe de Frank-Starling est contrôlée à chaque cycle ou, en d'autres termes, la réactivité du patient est contrôlée à chaque cycle avant d'introduire du fluide dans le corps du patient. Ce pilotage haute fréquence (N fois supérieure à la fréquence cardiaque) assure une adéquation optimale entre l'écoulement pulsatif et les besoins physiologiques du patient.

D'autres caractéristiques avantageuses de l'appareil objet de l'invention sont listées ci-dessous. Chacune de ces caractéristiques supplémentaires peut être considérée seule ou en combinaison avec les caractéristiques remarquables définies ci-dessus. Chacune de ces caractéristiques supplémentaires contribue, le cas échéant, à la résolution de problèmes techniques spécifiques définis plus avant dans la description et auxquels ne participent pas nécessairement les caractéristiques remarquables définies ci-dessus. Ces caractéristiques supplémentaires peuvent faire l'objet, le cas échéant, d'une ou plusieurs demandes de brevet divisionnaires :

Selon un mode de réalisation, l'unité de commande est configurée pour calculer la longueur de déplacement $L_i$ selon la formule suivante :

$$L_i = L_{i-1} + (\Pi_i\text{-}PR_i\text{-}\Delta P_{cor}) \times \frac{\Delta t^2}{S \times \mu}$$

Où

- $L_{i-1}$ est la longueur de déplacement sur le cycle $C_{i-1}$ ;
- $\Pi_i$ est la pression de consigne sur le cycle $C_i$ ;
- $PR_i$ est la pression réelle du fluide mesurée dans la chambre sur le cycle $C_i$ ;
- $\Delta P_{cor}$ est une valeur de pression corrective,
- S est la section équivalente de la surface de l'organe en contact avec le fluide,
- $\mu$ est une constante.

Selon un mode de réalisation, le dispositif comporte une canule d'éjection implantable dans le corps humain et connectée fluidiquement à la chambre, et dans lequel l'unité de commande est configurée pour calculer la longueur de déplacement $L_i$ selon la formule suivante :

$$L_i = L_{i-1} + (\Pi_i\text{-}PR_i) \times \frac{\Delta t^2}{S \times \mu}$$

Où

- $L_{i-1}$ est la longueur de déplacement sur le cycle $C_i$;
- $\Pi_i$ est la pression de consigne sur le cycle $C_i$ ;
- $PR_i$ est la pression réelle du fluide mesurée au niveau de la canule d'éjection $_{(16)}$ ;
- S est la section équivalente de la surface de l'organe en contact avec le fluide,
- $\mu$ est une constante.

Selon un mode de réalisation, l'unité de commande est configurée pour générer, pour chaque phase d'éjection, une courbe de pression de consigne selon la loi de Franck-Starling, la valeur de $\Pi_i$ correspondant à la valeur de la pression sur ladite courbe sur le cycle $C_i$.

Selon un mode de réalisation, la courbe de pression de consigne selon la loi de Franck-Starling est identique pour chaque phase d'éjection.

Selon un mode de réalisation, la courbe de pression de consigne selon la loi de Franck-Starling est redéfinie à chaque phase d'éjection ou redéfinie à intervalles de temps régulier en fonction de valeurs de pression artérielle mesurées à une fréquence pré-paramétrée, au niveau d'une extrémité du corps du patient.

Selon un mode de réalisation, l'unité de commande est configurée pour calculer, à chaque cycle $C_i$, la pression de consigne $\Pi_i$ selon la formule suivante :

$$\Pi_i = (PM_s - K_s \cdot VE_{i-1} - R_s \cdot Q_{i-1})$$

Où :

- PM$_S$ est la pression maximale systolique dont la valeur est pré-paramétrée et/ou réglée ;

- K$_S$ est un coefficient compris entre 0,9 et 1,1 ;

- VE$_{i-1}$ est le volume de fluide mis en mouvement dans la chambre pendant la phase d'éjection au cycle précédent C$_{i-1}$ ;

- R$_S$ est un coefficient compris entre 0,4 et 0,6 ;

- Q$_{i-1}$ est le débit de fluide éjecté de la chambre au cycle précédent C$_{i-1}$.

Selon un mode de réalisation, dans lequel le nombre N de cycles C$_i$ est compris entre 45 et 55, et la durée Δt de chaque cycle C$_i$ est compris entre 0,004 s et 0,006 s.

Selon un mode de réalisation, l'unité de commande est configurée pour dissocier les phases d'aspiration et les phases d'éjection du fluide de sorte que durant un cycle cardiaque artificiel, le volume aspiré lors de la phase d'aspiration est différent du volume éjecté lors de phases d'éjection.

Selon un mode de réalisation :

- le premier cycle C$_1$ coïncide avec la systole naturelle, et démarre à l'instant où est détecté un complexe QRS d'un signal ECG mesuré sur le patient, ou

- le premier cycle C$_1$ est décalé par rapport à la systole naturelle et démarre après un décalage compris entre 0,2 et 0,3 seconde de l'instant où est détecté un complexe QRS d'un signal ECG mesuré sur le patient.

## Brève description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :

[Fig. 1] est une vue schématique du dispositif d'assistance ou de suppléance circulatoire temporaire du cœur d'un patient selon l'invention.

[Fig. 2] est une vue schématique d'un système de pompage pouvant être utilisé dans un dispositif selon l'invention.

[Fig. 3] illustre le séquençage du déplacement de l'organe durant une phase d'éjection.

[Fig. 4] illustre une courbe de pression de consigne selon la loi de Franck-Starling.

## Description des modes de réalisation.

Le dispositif objet de l'invention est destiné à être utilisé lors d'une situation hémodynamique dégradée menaçant directement le pronostic vital d'un patient 12 (par exemple avec une pression de perfusion - PF - tissulaire inférieure à 50 mm Hg). Il permet d'assister ou de suppléer le cœur 13 du patient 12.

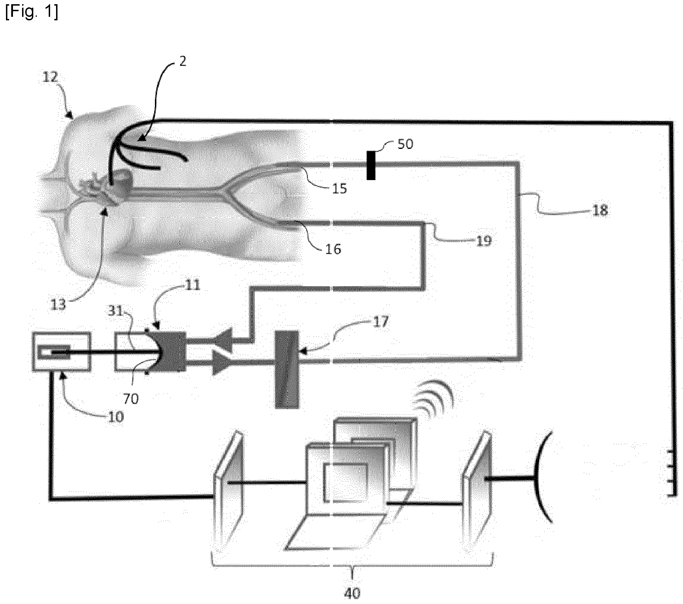

En se référant à la figure 1, le dispositif objet de l'invention comporte un système de pompage permettant de suppléer partiellement ou totalement le muscle cardiaque en aspirant une quantité suffisante de sang durant une phase de diastole artificielle et en la réinjectant durant une phase de systole artificielle.

Le tableau 1 ci-dessous illustre un exemple de séquencement de ces phases d'aspiration et d'éjection :

[Table 1]

| Cœur naturel | Dispositif objet de l'invention |
|---|---|
| Systole naturelle | Aspiration |
| Début diastole naturelle | Ejection |
| Suite diastole naturelle | Aspiration |

Le séquencement de ces phases d'aspiration et d'éjection est décrit plus en détail dans la suite de la description.

Selon un mode de réalisation, l'opérateur introduit une canule d'admission 15 (par ex. 21 ou 23 French ou « Fr », le FRENCH représentant 1/3 de millimètre) apte à prélever le sang dans le système veineux du patient 12 et une canule d'éjection 16 (par ex. 17 ou 19 French) apte à injecter le sang dans le système artériel dudit patient 12. Les canules d'admission 15 et d'éjection 16 que l'on utilise habituellement sur le marché de la circulation extra-corporelle (CEC, ECMO, ECLS) sont compatibles avec l'invention. On utilise préférentiellement des canules armées afin d'éviter un collapse d'aspiration et/ou une plicature desdites canules qui engendrerait une réduction du flux.

L'opérateur peut effectuer :

- préférentiellement une mise en place cœur droit / cœur gauche, de type ECMO veino-artériel, en cas de défaillance du cœur droit et du cœur gauche, en positionnant la canule d'admission 15 au niveau d'une veine cave et la canule d'éjection 16 au niveau de l'aorte. Dans ce dernier cas, les poumons sont également court-circuités et un système d'oxygénation 17 (comportant préférentiellement un échangeur thermique), est placé dans le circuit de dérivation pour éliminer le $CO_2$ du sang et le charger en $O_2$ avant de le réinjecter dans l'organisme. Ce système d'oxygénation 17 est avantageusement disposé après le réservoir 11, soit sur la portion d'éjection 18 du circuit de dérivation ;

- éventuellement une mise en place cœur droit / cœur droit pour une assistance partielle en cas de défaillance du cœur droit, en positionnant la canule d'admission 15 au niveau d'une veine cave et la canule d'éjection 16 au niveau de l'artère pulmonaire.

Les canules 15, 16 peuvent être mises en place par voie percutanée, dans une salle de cathétérisme cardiaque et/ou vasculaire ou dans une unité de réanimation ou par une unité UMAC SAMU (Unité Mobile d'Assistance Circulatoire pour UMAC et Service d'Aide Médical Urgence pour SAMU), en les introduisant par un vaisseau sanguin périphérique et en les amenant à proximité du cœur 13, au niveau des veines ou des artères visées. Elles peuvent également être mises en place par voie chirurgicale dans un bloc chirurgical, implantation mixte ponction percutanée et ouverture chirurgicale des vaisseaux.

Ces canules 15, 16 sont reliées au système de pompage par des tubes du type cathéter, aussi compatibles avec ceux que l'on utilise habituellement pour une CEC (par exemple de calibre 3/8ème) pour former d'une part la portion d'admission 19 et la portion d'éjection 18 du circuit de dérivation.

Le dispositif comporte une chambre 11 formant réservoir (équivalent à un ventricule externe artificiel) et permettant de stocker momentanément un volume de fluide (ex : sang, substitut sanguin, sang + substitut sanguin). Un actionneur linéaire 10 est configuré pour déplacer en translation un organe 70 dans la chambre 11 de manière à provoquer un écoulement de fluide pulsatif apte à soutenir l'activité du cœur 13. Cet écoulement est caractérisé par une succession de phases d'aspiration et de phases d'éjection du fluide.

À titre d'exemple et pour donner un ordre de grandeur, pour un coeur battant à 70 bpm (battement par minute), le débit moyen est d'environ 5 l/mn en éjection et en admission. L'éjection est brève (fort débit), l'aspiration est plus lente (environ deux fois moins de débit). La volémie type est environ 70 $cm^3$ ou 70 ml par battement. En 1 min (70 battements), le volume pompé est de : 70x70 ml=4900 ml, soit 4,9 l. Les 70 ml sont éjectés entre 0,2 s et 0,35 s (selon le patient), de sorte que le débit instantané maximal à l'éjection est de 270 ml/s ou 16 l/min max. Ce débit est à peu près deux fois moindre en aspiration. Grâce au dispositif selon l'invention, on peut augmenter (ou éventuellement réduire) les quantités de sang aspiré/éjecté pour correspondre au plus près au fonctionnement réel d'un cœur et aux besoins de l'organisme.

Selon un mode de réalisation, l'actionneur 10 est du type moteur linéaire ou tout autre moyen équivalent (par exemple un vérin) permettant de déplacer l'organe 70. Selon un mode de réalisation, l'organe 70 est une membrane fixée, sur sa périphérie, à la paroi interne de la chambre 11. Elle est avantageusement réalisée dans un matériau flexible et élastique, élastomère ou autre. La membrane 70 loge un insert central, non visible sur les figures annexées, doté d'un bras d'actionnement formant piston 31, ce piston étant fixé à l'actionneur 10 grâce à un moyen mécanique d'engagement, de fixation ou de connexion. Selon un autre mode de réalisation, l'organe 70 se présente sous la forme d'un piston du type décrit dans la demande de brevet précitée WO2006/016047.

Selon un mode de réalisation, l'actionneur 10 est configuré pour exécuter les commandes de déplacement à haute vélocité afin d'ajuster le mouvement réel du fluide auxdites commandes. On peut utiliser pour cela un moteur linéaire à faible constante de temps (avantageusement inférieure ou égale à 10 ms) et capable de générer une force importante (avantageusement supérieure ou égale à 500 N), auquel on peut adjoindre un système de bras de levier pour l'accélérer encore. Le fluide peut alors être brutalement mis en mouvement et arrêté au moment choisi. Le régime physique d'écoulement du fluide peut ainsi suivre de très près les signaux de commande.

Une unité de commande 40 est prévue pour piloter automatiquement l'actionneur 10 et contrôler le déplacement de l'organe 70. Cette unité 40 peut se présenter sous la forme d'un processeur, microprocesseurs, CPU (pour Central Processing Unit) intégré dans un ordinateur, un calculateur ou moyen analogue.

Chaque pas de déplacement de l'actionneur 10 correspond à un volume interne de la chambre 11. Cette correspondance entre le pas de l'actionneur 10 et le volume interne de la chambre 11 est stockée ou enregistrée dans une zone mémoire de l'unité 40. En contrôlant le déplacement de l'actionneur 10, on peut donc contrôler très précisément le volume de fluide aspiré et éjecté par le dispositif lors des phases successives d'aspiration et d'éjection.

Selon un mode de réalisation, un ou plusieurs capteurs 2 sont mis en place pour acquérir un signal électrocardiographique (signal ECG) qui correspond à une activité électrique du cœur 13. L'unité de commande 40 est configurée pour piloter le déplacement de l'actionneur 10 en prenant en compte des données d'entrée provenant de mesures de paramètres hémodynamiques du patient et notamment de ce signal ECG. Les données d'entrées peuvent provenir d'autres mesures de paramètres hémodynamiques, telles que la pression au niveau de la canule 15 et/ou de la canule 16, la saturation en oxygène dans le sang (mesurée au moyen d'un oxymètre de pouls), les variations de pression ou de volumes régnant dans un organe ou un segment de membre (mesurée au moyen d'un pléthysmographe), etc. Selon un mode préféré de réalisation, l'acquisition de ces données est réalisée en temps réel, à une fréquence rapide de 200 Hz.

## Systole artificielle

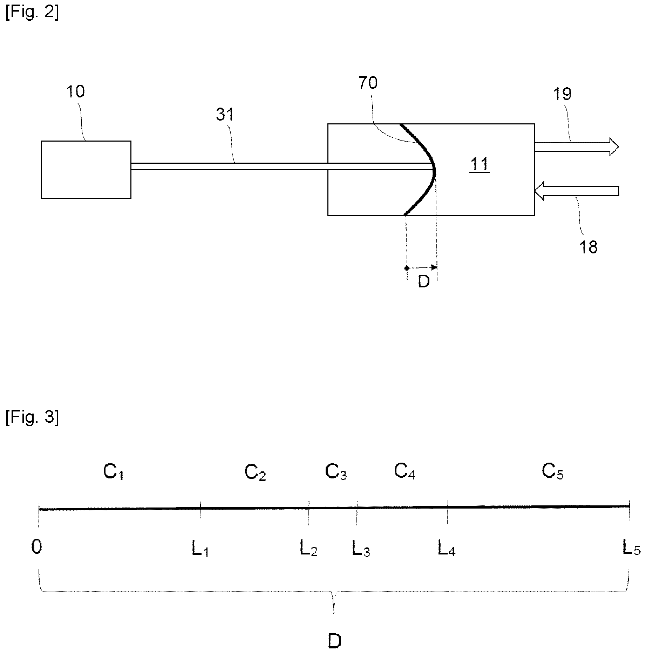

En se rapportant à la figure 2, durant chaque phase d'éjection du fluide hors de la chambre 11 (chaque systole artificielle), l'unité 40 déplace l'organe 70 d'une distance D. Cette distance D n'est pas nécessairement constante, mais peut être variable à chaque systole artificielle.

Selon un mode de réalisation, le déplacement D est séquencé en un nombre entier N de cycles $C_i$, avec N>1, chaque cycle ayant la même durée $\Delta t$. La figure 3 illustre à titre d'exemple explicatif un séquençage du déplacement D en 5 cycles Ci (N=5). Le nombre N est avantageusement compris entre 2 et 100, préférentiellement entre 10 et 80, et très préférentiellement entre 20 et 60. Selon un mode préféré de réalisation permettant d'obtenir un excellent compromis en termes de précision de calculs et de ressources informatiques nécessaires audits calculs, N est compris entre 45 et 55, préférentiellement égal à 50. La durée $\Delta t$ est avantageusement comprise entre 0,1 s (seconde) et 0,003 s, préférentiellement entre 0,004 s et 0,006 s, et très préférentiellement égale à 0.005 s. Ainsi, l'unité 40 définie à chaque cycle $C_i$ le besoin du patient 12 et contrôle précisément l'alimentation de l'organisme dudit patient selon ce même besoin.

La dynamique du battement maximale (systole) est de l'ordre de 0,3 s Selon un mode de réalisation, pour bien discrétiser cette dynamique, celle-ci est découpée au minimum en 20 étapes, soit un pas de temps maximum de 0,3/20=0,015 s, correspondant à 60 Hz de fréquence minimum. Un bon compromis est obtenu avec une marge de précision de 200 Hz, ce qui permet de piloter les mouvements de l'organe 70 avec une précision de 0,005 s. On obtient notamment ainsi un réglage optimal et précis des temps au 100$^{\text{ème}}$ de seconde.

Selon un mode de réalisation, le premier cycle $C_1$ coïncide avec la systole naturelle, et démarre par exemple à l'instant où est détecté le complexe QRS du signal ECG. Selon un autre mode de réalisation, le premier cycle $C_1$ est décalé par rapport à la systole naturelle d'un décalage de l'ordre de 0,25 seconde environ (soit entre 0,2 et 0,3 seconde) de l'instant où est détecté le complexe QRS du signal ECG. Ce décalage temporel, rendu possible par la haute cadence de calcul, permet de s'assurer que la valve aortique est parfaitement refermée lorsque la systole artificielle est initiée, de sorte que le ventricule est protégé de toute surcharge.

Sur chaque cycle $C_i$, l'unité 40 calcule une longueur de déplacement $L_i$ de l'organe 70 telle que $D = \sum_{i=1}^{N} Li$. Sur l'exemple de la figure 3, D=$L_1$+$L_2$+$L_3$+$L_4$+$L_5$, avec $L_1 \neq L_2 \neq L_3 \neq_4 \neq L_5$. Chaque longueur $L_i$ est calculée en fonction des données d'entrée provenant d'un ou plusieurs paramètres hémodynamiques du patient 12 mesurés pendant le cycle $C_i$ et d'une valeur de pression de consigne sur ledit cycle $C_i$. Dès que la longueur $L_i$ est calculée, l'unité 40 transmet une instruction à l'actionneur 10 pour déplacer l'organe 7) de ladite longueur.

Les paramètres hémodynamiques mesurés pendant le cycle $C_i$ peuvent être tout ou partie de ceux mentionnés précédemment (signal ECG, pression au niveau des canules 15, 16, la saturation en oxygène, variations de pression ou de volume, ...). Il y a donc une acquisition de ces paramètres dans chaque intervalle de temps $\Delta t$, ce qui permet un contrôle en temps réel de l'état de patient 12 et un pilotage correspondant de l'organe 70 qui est extrêmement précis sur la distance D.

En outre, sur chaque cycle $C_i$, l'unité 40 détermine une pression de consigne basée sur la loi de Franck-Starling.

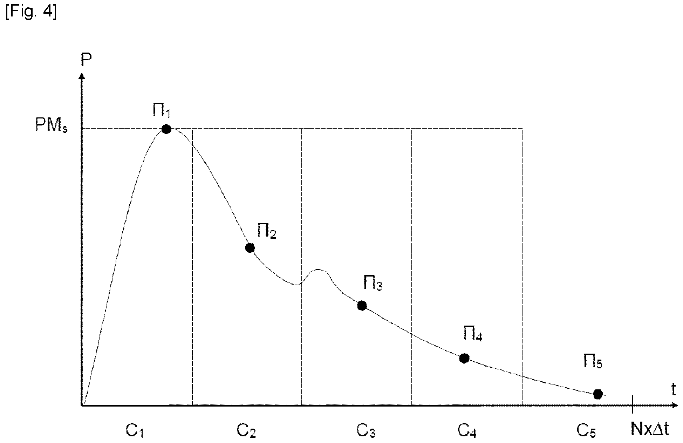

Selon un premier mode de réalisation, l'unité 40 génère, pour chaque phase d'éjection, une courbe de pression de consigne selon, au départ, la loi de Franck-Starling. La figure 4 illustre une courbe de variation de pression selon la loi de Frank-Starling, qui a typiquement une forme de courbe en cloche passant par une pression maximale PM$_S$. Cette courbe de consigne peut être identique pour chaque phase d'éjection ou, préférentiellement être redéfinie à chaque phase d'éjection ou à intervalle de temps régulier (par exemple toutes les 5 minutes ou toutes les 10 minutes), en fonction de l'état du patient notamment défini par les mesures de paramètres hémodynamiques et/ou d'un algorithme de calcul basé sur un modèle d'intelligence artificielle, tout en restant fondée sur la loi de Frank-Starling telle que revendiquée.

Avantageusement, la courbe de consigne est redéfinie à intervalles de temps régulier en fonction de valeurs de pression artérielle mesurées à une fréquence pré-paramétrée, au niveau d'une extrémité du corps du patient, par exemple au bout d'un doigt. Cette mesure de pression permet de renseigner sur l'état physiologique du patient 12 et permet de recaler la courbe de consigne. En effet, les résistances et compliances systémiques de l'organisme du patient évoluent au cours d'une journée et/ou en fonction d'un éventuel traitement médicamenteux dudit patient. L'écoulement pulsatif artificiel peut ainsi être adapté très simplement à ces variations. Si la pression mesurée à l'extrémité du corps du patient est inférieure à une valeur seuil (par exemple inférieure à $PM_S$x10%), alors la valeur de $PM_S$ (c.-à-d. le pic de la courbe en cloche) est augmentée. Inversement, si la pression mesurée à l'extrémité du corps du patient est supérieure à une valeur seuil (par exemple supérieure à $PM_S$x10%), alors la valeur de $PM_S$ est réduite. On s'assure ainsi que fluide pénètre profondément dans l'organisme et irrigue convenablement les organes du patient 12 durant toute la durée de l'assistance ou de la suppléance circulatoire.

La courbe de consigne est définie sur la période $N$x$\Delta t$, où $N$ est le nombre de cycles Ci et $\Delta t$ l'intervalle de temps de chaque cycle. À chaque cycle Ci correspond une valeur de la pression de consigne $\Pi_i$, laquelle valeur est utilisée par l'unité 40 comme donnée d'entrée pour le calcul de la longueur $L_i$. La pression du fluide éjecté suit ainsi au plus près cette courbe de consigne.

Selon un deuxième mode de réalisation, l'unité 40 calcule, à chaque cycle Ci, la pression de consigne $\Pi_i$ selon la formule [Math 1] basée sur la loi de Franck-Starling :

[Math 1]

$$\Pi_i = (PM_S - K_s \cdot VE_{i-1} - R_s \cdot Q_{i-1})$$

Où :

- $PM_S$ est la pression maximale systolique dont la valeur est pré-paramétrée et/ou réglée par le praticien ou automatiquement, par exemple égale à 140 mm.Hg ; comme expliqué précédemment, la valeur de $PM_S$ peut être redéfinie à intervalle de temps régulier en fonction de valeurs de pression artérielle mesurées à une fréquence pré-paramétrée, au niveau d'une extrémité du corps du patient ;

- $K_S$ est un coefficient systolique compris entre 0,9 et 1,1 ;

- $VE_{i-1}$ est le volume de fluide mis en mouvement dans la chambre 11, pendant la phase d'éjection, au cycle précédent $C_{i-1}$ ;

- $R_S$ est un coefficient exprimant la résistance circulatoire dans les artères, compris entre 0,4 et 0,6 ;

- $Q_{i-1}$ est le débit de fluide éjecté de la chambre 11 au cycle précédent $C_{i-1}$.

Selon un troisième mode de réalisation, la valeur de pression de consigne $\Pi_i$ est déterminée au moyen d'un algorithme de calcul basé sur un modèle d'intelligence artificielle.

Selon un mode de réalisation, la longueur $L_i$ est déterminée selon la formule [Math 2] suivante :

[Math 2]

$$L_i = L_{i-1} + (\Pi_i \text{-} PR_i \text{-} \Delta P_{cor}) \times \frac{\Delta t^2}{S \times \mu}$$

Où

- $L_{i-1}$ est la longueur de déplacement sur le cycle précédent $C_{i-1}$ ;

- $\Pi_i$ est la pression de consigne sur le cycle $C_i$ ;

- $PR_i$ est la pression réelle du fluide mesurée dans la chambre 11 sur le cycle $C_i$ ;

- $\Delta P_{cor}$ est une valeur de pression corrective,

- $S$ est la section équivalente de la surface de l'organe 70 en contact avec le fluide,

- $\mu$ est une constante (correspondant à l'inertance sanguine).

La pression $PR_i$ est avantageusement mesurée au moyen d'un capteur de pression installé dans la chambre 11. Pour conférer davantage de sécurité au dispositif, cette mesure de pression dans la chambre 11 peut être corrélée à une ou plusieurs autres mesures de pression par capteur installé au niveau de la canule 15 et/ou de la canule 16. Si l'un de ces différents capteurs se dérègle ou devient défectueux, il est alors possible d'arrêter la méthode de régulation en pression selon l'invention et/ou de remplacer ladite méthode par une autre méthode de régulation (par exemple une régulation en volume).

La valeur de pression corrective $\Delta P_{cor}$ peut être fixe ou variable à chaque cycle $C_i$. Elle est par exemple comprise entre 10 mm.Hg et 50 mm.Hg (1 mm.Hg = 133,322 Pa). Compte tenu notamment des longueurs et de l'élasticité des tubes reliant la chambre 11 à la canule 16, des éventuels appareils 17 installés dans le circuit de dérivation, de la viscosité du fluide, de l'élasticité de l'aorte, la pression $PR_i$ mesurée dans la chambre 11 peut différer de la pression réelle du fluide dans l'aorte. La pression corrective $\Delta P_{cor}$ apporte ce correctif.

La valeur de $\mu$ représente l'inertie de la colonne de fluide déplacé, laquelle valeur est par exemple comprise entre $10^6$ kg/m$^4$ et $5.10^6$ kg/m$^4$.

Selon un autre mode de réalisation, la pression $PR_i$ correspond à la pression réelle du fluide mesurée au niveau de la canule d'éjection 16 sur le cycle $C_i$. Dans ce cas, de bons résultats sont obtenus même en n'appliquant pas de correction de pression ($\Delta P_{cor}=0$) de sorte que la longueur $L_i$ peut être déterminée selon la formule [Math 3] suivante :

[Math 3]

$$L_i = L_{i-1} + (\Pi_i - PR_i) \times \frac{\Delta t^2}{S \times \mu}$$

Où

- $L_{i-1}$ est la longueur de déplacement sur le cycle précédent $C_{i-1}$ ;

- $\Pi_i$ est la pression de consigne sur le cycle $C_i$ ;

- $PR_i$ est la pression réelle du fluide mesurée au niveau de la canule 16 sur le cycle $C_i$ ;

- S est la section équivalente de la surface de l'organe 70 en contact avec le fluide,

- $\mu$ est une constante (identique à celle de la formule [Math 2]).

Grâce à l'apport de l'invention, le fluide expulsé durant la systole artificielle pénètre profondément dans l'organisme du patient 12, selon ses besoins physiologiques, afin d'irriguer les différents organes. Le profil débit/pression du fluide éjecté se combine avec les compliances artérielles pour que l'onde systolique se propage correctement dans l'organisme.

Les meilleurs résultats sont obtenus lorsque l'acquisition des paramètres hémodynamiques, des pressions réelles, la détermination de la pression de consigne, l'élaboration de la commande de déplacement $L_i$ et son exécution par l'unité 40 sont réalisées pendant le cycle $C_i$. Toutefois, selon un autre mode de réalisation donnant tout de même des résultats convenables, l'acquisition des paramètres hémodynamiques et des pressions réelles, la détermination de la pression de consigne, l'élaboration de la commande de déplacement $L_i$ et son exécution par l'unité 40 peuvent être réalisées pendant un ou plusieurs cycles précédents le cycle $C_i$ et proches dudit cycle $C_i$. Par proche, on entend trois ou quatre cycles précédents le cycle $C_i$ durant lequel est exécuté la commande de déplacement. Par exemple, l'acquisition des paramètres hémodynamiques peut être réalisée au cycle $C_{i-3}$, la pression réelle du fluide également mesuré sur le cycle $C_{i-3}$, le calcul de la pression de consigne $\Pi_{i-2}$ réalisé durant le cycle $C_{i-2}$, l'élaboration de la commande de déplacement Li réalisée sur le cycle $C_{i-1}$ pour être exécutée sur le cycle $C_i$. L'homme du métier comprendra que d'autres séquencements dans le temps sont envisageables.

**Diastole artificielle**

L'exécution de la diastole artificielle est indépendante celle de la systole artificielle. Selon un mode de réalisation, l'admission du sang dans la chambre 11 (diastole artificielle) est réalisée tout le temps du cycle cardiaque naturel, mis à part le temps dédié à la systole artificielle.

Selon un mode de réalisation, le temps de la diastole artificielle est d'environ 0,5 seconde (environ 1/3 de la systole artificielle), l'organe 70 se déplaçant d'environ 1 cm.

Selon un mode de réalisation, le débit de fluide aspiré dans la chambre 11 pendant la diastole artificielle est important, voire très important, d'au moins 2 à 3 voire 5 litres par minute. Compte tenu de ce débit élevé d'aspiration, le risque de collabage de la veine cave du patient 12 est important, voire très important. Il est donc particulièrement intéressant de gérer le plus finement possible l'aspiration, c'est-à-dire de pouvoir moduler ou modifier l'aspiration du fluide prélevé au niveau de la veine cave du patient afin d'éviter tout risque de collabage.

Pour atteindre cet objectif, un capteur de pression est avantageusement installé dans la portion d'admission 19 (au niveau de la canule d'admission 15 ou entre ladite canule et la chambre 11). Ce capteur de pression présente une constante de temps de mesure inférieure à 20 millisecondes, avantageusement inférieure à 10 millisecondes, afin de détecter très rapidement et très régulièrement dans le temps la pression et toute modification de cette dernière. L'unité 40 peut ainsi moduler ou arrêter l'aspiration lorsque la pression détectée par ledit capteur atteint une valeur de pression seuil et/ou que la pression augmente/diminue au-delà ou en dessous d'une pente d'accélération/décélération de pression seuil. Selon un mode de réalisation, la valeur de pression seuil est comprise entre 6 mm.Hg et 15 mm.Hg, préférentiellement égale ou sensiblement égale à 12 mm.Hg. On évite tout risque de collabage au niveau de la veine cave, grâce aux valeurs seuils (absolue et/ou de pente d'accélération) et à la détection continue et rapide (fréquence élevée) de la pression dans la portion d'admission 19.

L'unité 40 dispose de moyens informatiques dans lesquels on a programmé deux types d'alertes relativement à la pression détectée dans la portion d'admission 19. Tout d'abord, un niveau seuil en valeur absolue de pression qui, s'il est atteint, déclenche une modification du pompage du sang, classiquement en diminuant le débit de pompage. Ensuite, la deuxième alerte consiste en l'accélération/décélération de la pression, entre deux mesures de pression dans le temps : à nouveau, si cette pente de seuil (d'accélération/décélération) est atteinte, l'aspiration du sang est modifiée, classiquement en la diminuant ou en la stoppant. Concernant la pente de seuil, le programme prédictif de l'évolution de la pression utilise classiquement le nombre de Reynold ou encore l'onde de Reynold. Le nombre de Reynolds correspond à un nombre sans dimension qui est utilisé en mécanique des fluides. Cette grandeur permet de caractériser un écoulement, en particulier la nature de son régime. Il est ainsi possible de savoir si un écoulement est laminaire, transitoire ou turbulent.

À chaque pulsation, un ordre de grandeur du volume de fluide aspiré est de l'ordre de 50 ml (millilitre) mais cela peut monter à 100 ml, ceci dépendant du patient. Un débit cardiaque naturel est compris entre 2,5 et 4,5 l/mn/m$^2$ (litre par minute par mètre carré). Autrement dit, plus la surface d'un corps humain (et donc son poids) est importante, plus la circulation sanguine est également importante. En pratique, pour ajuster le poids ou le volume de fluide à aspirer à chaque pulsation, le médecin considère le poids du patient 12 et en déduit le volume à chaque pulsation.

Pour un cœur battant à 70 bpm (battement par minute), la durée d'aspiration est d'environ 0,56 seconde de sorte qu'un débit normal est d'environ 90 ml/s (millilitre par seconde par systole) soit 5 l/mn (litre par minute). Or, dans la pratique, le médecin s'oriente sur un débit moyen de 3 ou 4 l/mn. Grâce au dispositif selon l'invention, on peut augmenter (ou éventuellement réduire) les quantités de sang aspiré pour correspondre au plus près au fonctionnement réel d'un cœur, sans risquer de collabage au niveau de la veine cave.

A titre d'exemple, pour la valeur de seuil de pression (première alerte) et la valeur de seuil de pente ou d'accélération de la pression (deuxième alerte) :

- si la pente ou l'accélération de la pression mesurée est inférieure de 50% à ce qu'elle devrait être au cours d'un cycle d'aspiration, alors cela signifie qu'il y a un risque imminent de collabage, et l'unité 40 réduit immédiatement le débit aspiré ;

- si la pression absolue descend en dessous de la valeur de pression de seuil de 12 mm.Hg, alors l'unité 40 cesse immédiatement le pompage pour ce cycle d'aspiration, car le risque de collabage est très/trop significatif. Le pompage redémarre au cycle d'aspiration suivant.

Selon un mode de réalisation, sur un cycle cardiaque artificiel, le volume aspiré lors de la diastole artificielle correspond au volume éjecté lors de la systole artificielle. Toutefois, selon l'état du patient (ex : hypertension, état du système veineux, obstruction artérielle, viscosité du sang, ...), le volume aspiré lors de la diastole artificielle peut être différent du volume éjecté lors de la systole artificielle. Par exemple, l'unité 40 peut contrôler le déplacement de l'organe 70 de sorte que durant la diastole artificielle, un volume suffisant de fluide soit aspiré pour assurer une bonne circulation dans le corps du patient, cette aspiration pouvant être lente (ex : 70% du cycle cardiaque) et à faible dynamique pour éviter les risques de collabage. Pour un patient souffrant d'hypertension, il peut être avantageux de ne pas réinjecter la totalité de ce volume aspiré pour éviter de contribuer à une surpression de l'organisme du patient. Par exemple encore, pour un patient 12 souffrant d'obstruction artérielle, l'unité 40 peut être configurée pour effectuer deux diastoles artificielles sur deux cycles cardiaques successifs (sans systole artificielle), pour commander la systole artificielle sur le cycle suivant, avec un profil de débit/pression optimal pour que l'onde systolique se propage profondément dans l'organisme du patient 12 malgré l'obstruction artérielle.

De façon plus générale, la configuration de l'unité 40 permet de dissocier les diastoles artificielles et les systoles artificielles : l'aspiration du fluide peut être indépendante du régime d'éjection dans l'aorte, ce qui correspond à la réalité de l'organisme du patient 12. La chambre 11 combinée au déplacement contrôlé de l'organe 70, assure une fonction d'assignation d'équilibre diastolique (AED) : le volume moyen de la chambre 11 permet de régler le volume de fluide dans l'organisme du patient 12, et donc la pression sanguine moyenne dans ledit organisme.

Les meilleurs résultats pour obtenir une synchronisation optimale entre la circulation artificielle et les besoins de l'organisme du patient 12 s'obtiennent notamment par la maîtrise des deux éléments suivants :

- Un traitement rapide des mesures (avec préférentiellement une fréquence d'acquisition rapide de 200 Hz ou de cet ordre de fréquence) pour envoyer en temps réel les commandes à l'actionneur 10.
- Une exécution des commandes à haute vélocité pour ajuster le mouvement réel du fluide aux commandes.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de l'esprit et de la portée de l'invention.

En outre, une ou plusieurs caractéristiques exposées seulement dans un mode de réalisation peuvent être combinées avec une ou plusieurs autres caractéristiques exposées seulement dans un autre mode de réalisation. De même, une ou plusieurs caractéristiques exposées seulement dans un mode de réalisation peuvent être généralisées aux autres modes de réalisation, même si ce ou ces caractéristiques sont décrites seulement en combinaison avec d'autres caractéristiques.

En tout état de cause, dans les revendications, tout signe de référence entre parenthèses ne saurait être interprété comme une limitation de la revendication.

Un autre aspect non couvert par l'invention et les revendications, concerne un dispositif d'assistance ou de suppléance circulatoire temporaire du cœur d'un patient, qui n'est pas extracorporelle mais implantable et comprenant :

- un actionneur linéaire configuré pour déplacer en translation un organe dans une chambre de manière à provoquer un écoulement de fluide pulsatif apte à soutenir l'activité du cœur dudit patient, lequel écoulement est caractérisé par une succession de phases d'aspiration et de phases d'éjection du fluide,
- une unité de commande configurée pour piloter l'actionneur et contrôler le déplacement de l'organe, lequel pilotage est réalisé en prenant en compte des données d'entrée provenant de mesures de paramètres hémodynamiques du patient, durant chaque phase d'éjection ladite unité déplaçant l'organe d'une distance D.

L'unité de commande est configurée pour :

- séquencer le déplacement D en un nombre entier N de cycles $C_i$, avec N>1, chaque cycle ayant la même durée $\Delta t$,

- sur chaque cycle $C_i$, calculer une longueur de déplacement $L_i$ de l'organe telle que $D = \sum_{i=1}^{N} Li$, laquelle longueur $L_i$ est calculée en fonction des données d'entrée provenant :

   ◦ d'un ou plusieurs paramètres hémodynamiques du patient mesurés pendant le cycle $C_i$, ou pendant un ou plusieurs cycles précédents ledit cycle $C_i$ et proches dudit cycle $C_i$,
   ◦ de la valeur d'une pression de consigne $\Pi_i$ basée sur la loi de Franck-Starling,

- sur chaque cycle $C_i$, transmettre une instruction à l'actionneur pour déplacer l'organe de la longueur $L_i$ calculée.

Les autres caratéristiques revendiquées et décrites précédemment s'appliquent à ce mode de réalisation non couvert par l'invention.

## Revendications

1. Dispositif d'assistance ou de suppléance circulatoire extracorporelle temporaire du cœur (13) d'un patient (12), comprenant :

   - un actionneur linéaire (10) configuré pour déplacer en translation un organe (70) dans une chambre (11) de manière à provoquer un écoulement de fluide pulsatif apte à soutenir l'activité du cœur dudit patient, lequel écoulement est **caractérisé par** une succession de phases d'aspiration et de phases d'éjection du fluide,

- une unité de commande (40) configurée pour piloter l'actionneur (10) et contrôler le déplacement de l'organe (70), lequel pilotage est réalisé en prenant en compte des données d'entrée provenant de mesures de paramètres hémodynamiques du patient (12), durant chaque phase d'éjection ladite unité déplaçant l'organe (70) d'une distance D,

**caractérisé en ce que** l'unité de commande (40) est configurée pour :

- séquencer le déplacement de distance D en un nombre entier N de cycles $C_i$, avec N>1, chaque cycle ayant la même durée $\Delta t$,

- sur chaque cycle $C_i$, calculer une longueur de déplacement $L_i$ de l'organe (70) telle que $D = \sum_{i=1}^{N} Li$, laquelle longueur $L_i$ est calculée en fonction des données d'entrée provenant :

  ○ d'un ou plusieurs paramètres hémodynamiques du patient (12) mesurés pendant le cycle $C_i$, ou pendant un ou plusieurs cycles précédents ledit cycle $C_i$ et proches dudit cycle $C_i$,
  ○ de la valeur d'une pression de consigne $\Pi_i$ basée sur la loi de Franck-Starling,

- sur chaque cycle $C_i$, transmettre une instruction à l'actionneur (10) pour déplacer l'organe (70) de la longueur $L_i$ calculée.

2. Dispositif selon la revendication 1, dans lequel l'unité de commande (40) est configurée pour calculer la longueur de déplacement $L_i$ selon la formule suivante :

$$L_i = L_{i-1} + (\Pi_i\text{-}PR_i\text{-}\Delta P_{cor}) \times \frac{\Delta t^2}{S \times \mu}$$

Où

- $L_{i-1}$ est la longueur de déplacement sur le cycle $C_{i-1}$ ;
- $\Pi_i$ est la pression de consigne sur le cycle $C_i$ ;
- $PR_i$ est la pression réelle du fluide mesurée dans la chambre (11) sur le cycle $C_i$ ;
- $\Delta P_{cor}$ est une valeur de pression corrective,
- S est la section équivalente de la surface de l'organe (70) en contact avec le fluide,
- $\mu$ est une constante.

3. Dispositif selon la revendication 1, comprenant en outre une canule d'éjection (16) implantable dans le corps humain et connectée fluidiquement à la chambre (11), et dans lequel l'unité de commande (40) est configurée pour calculer la longueur de déplacement $L_i$ selon la formule suivante :

$$L_i = L_{i-1} + (\Pi_i\text{-}PR_i) \times \frac{\Delta t^2}{S \times \mu}$$

Où

- $L_{i-1}$ est la longueur de déplacement sur le cycle $C_i$ ;
- $\Pi_i$ est la pression de consigne sur le cycle $C_i$ ;
- $PR_i$ est la pression réelle du fluide mesurée au niveau de la canule d'éjection (16) ;
- S est la section équivalente de la surface de l'organe (70) en contact avec le fluide,
- $\mu$ est une constante.

4. Dispositif selon l'une des revendications précédentes, dans lequel l'unité de commande (40) est configurée pour générer, pour chaque phase d'éjection, une courbe de pression de consigne selon la loi de Franck-Starling, la valeur de $\Pi_i$ correspondant à la valeur de la pression sur ladite courbe sur le cycle $C_i$.

5. Dispositif selon la revendication 4, dans lequel la courbe de pression de consigne selon la loi de Franck-Starling est identique pour chaque phase d'éjection.

6. Dispositif selon la revendication 4, dans lequel la courbe de pression de consigne selon la loi de Franck-Starling est redéfinie à chaque phase d'éjection ou redéfinie à intervalles de temps régulier en fonction de valeurs de pression artérielle mesurées à une fréquence pré-paramétrée, au niveau d'une extrémité du corps du patient.

7. Dispositif selon l'une des revendications 1 à 3, dans lequel l'unité de commande (40) est configurée pour calculer, à chaque cycle $C_i$, la pression de consigne $\Pi_i$ selon la formule suivante :

$$\Pi_i = (PM_s - K_s \cdot VE_{i-1} - R_s \cdot Q_{i-1})$$

Où :

- $PM_S$ est la pression maximale systolique dont la valeur est pré-paramétrée et/ou réglée ;
- $K_S$ est un coefficient compris entre 0,9 et 1,1 ;
- $VE_{i-1}$ est le volume de fluide mis en mouvement dans la chambre (11) pendant la phase d'éjection au cycle précédent $C_{i-1}$ ;
- $R_S$ est un coefficient compris entre 0,4 et 0,6 ;
- $Q_{i-1}$ est le débit de fluide éjecté de la chambre (11) au cycle précédent $C_{i-1}$.

8. Dispositif selon l'une des revendications précédentes, dans lequel le nombre N de cycles $C_i$ est compris entre 45 et 55, et la durée $\Delta t$ de chaque cycle $C_i$ est compris entre 0,004 s et 0,006 s.

9. Dispositif selon l'une des revendications précédentes, dans lequel l'unité de commande (40) est configurée pour dissocier les phases d'aspiration et les phases d'éjection du fluide de sorte que durant un cycle cardiaque artificiel, le volume aspiré lors de la phase d'aspiration est différent du volume éjecté lors de phases d'éjection.

10. Dispositif selon l'une des revendications précédentes, dans lequel :

- le premier cycle $C_1$ coïncide avec la systole naturelle, et démarre à l'instant où est détecté un complexe QRS d'un signal électrocardiographique ECG mesuré sur le patient (12), ou
- le premier cycle $C_1$ est décalé par rapport à la systole naturelle et démarre après un décalage compris entre 0,2 et 0,3 seconde de l'instant où est détecté un complexe QRS d'un signal électrocardiographique ECG mesuré sur le patient (12).


**Patentansprüche**

1. Vorrichtung zur temporären extrakorporalen Kreislaufunterstützung oder Ersatzversorgung des Herzens (13) eines Patienten (12), umfassend:

- ein Linearstellglied (10), das konfiguriert ist, um ein Organ (70) in einer Kammer (11) zu verschieben, um eine pulsierende Fluidströmung zu bewirken, die imstande ist, die Aktivität des Herzens des Patienten zu unterstützen, wobei die Strömung durch eine Abfolge von Ansaugphasen und Ausstoßphasen des Fluids gekennzeichnet ist,
- eine Steuereinheit (40), die konfiguriert ist, um das Stellglied (10) anzusteuern und die Verschiebung des Organs (70) zu steuern, wobei die Ansteuerung unter Berücksichtigung der aus Messungen hämodynamischer Parameter des Patienten (12) während jeder Ausstoßphase stammenden Eingangsdaten erfolgt, wobei die Einheit das Organ (70) um einen Abstand D verschiebt,

**dadurch gekennzeichnet, dass die** Steuereinheit (40) konfiguriert ist, um:

- die Verschiebung um den Abstand D in einer ganzen Anzahl N von Zyklen $C_i$ zu sequenzieren, wobei N>1 ist, wobei jeder Zyklus die gleiche Dauer $\Delta t$ aufweist,

- bei jedem Zyklus $C_i$ eine Verschiebungslänge $L_i$ des Organs (70) zu berechnen, sodass $D = \sum_{i=1}^{N} Li$ , wobei die Länge $L_i$ in Abhängigkeit von den Eingangsdaten berechnet wird, aus:

○ einem oder mehreren hämodynamischen Parametern des Patienten (12), die während des Zyklus $C_i$ oder während eines oder mehrerer Zyklen vor dem Zyklus $C_i$ und nahe dem Zyklus $C_i$ gemessen wurden,
○ dem Wert eines Solldrucks $\Pi_i$ basierend auf dem Frank-Starling-Gesetz,

- bei jedem Zyklus $C_i$ eine Anweisung an das Stellglied (10) zu übertragen, um das Organ (70) um die berechnete Länge $L_i$ zu verschieben.

2. Vorrichtung nach Anspruch 1, wobei die Steuereinheit (40) konfiguriert ist, um die Verschiebungslänge $L_i$ gemäß der folgenden Formel zu berechnen:

$$L_i = L_{i-1} + (\Pi_i \text{-} PR_i \text{-} \Delta P_{cor}) \times \frac{\Delta t^2}{S \times \mu}$$

Wobei

- $L_{i-1}$ die Verschiebungslänge im Zyklus $C_{i-1}$ ist;
- $\Pi_i$ der Solldruck im Zyklus $C_i$ ist;
- $PR_i$ der gemessene Ist-Druck in der Kammer (11) im Zyklus $C_i$ ist;
- $\Delta P_{cor}$ ein Korrekturdruckwert ist,
- S der äquivalente Querschnitt der Oberfläche des mit dem Fluid in Berührung kommenden Organs (70) ist,
- $\mu$ eine Konstante ist.

3. Vorrichtung nach Anspruch 1, weiter umfassend eine in den menschlichen Körper implantierbare und fluidisch mit der Kammer (11) verbundene Ausstoßkanüle (16), und wobei die Steuereinheit (40) konfiguriert ist, um die Verschiebungslänge $L_i$ gemäß der folgenden Formel zu berechnen:

$$L_i = L_{i-1} + (\Pi_i \text{-} PR_i) \times \frac{\Delta t^2}{S \times \mu}$$

Wobei

- $L_{i-1}$ die Verschiebungslänge im Zyklus $C_i$ ist;
- $\Pi_i$ der Solldruck im Zyklus $C_i$ ist;
- $PR_i$ der gemessene Ist-Druck im Bereich der Ausstoßkanüle (16) ist;
- S der äquivalente Querschnitt der Oberfläche des mit dem Fluid in Berührung kommenden Organs (70) ist,
- $\mu$ eine Konstante ist.

4. Vorrichtung nach einem der vorstehenden Ansprüche, wobei die Steuereinheit (40) konfiguriert ist, um für jede Ausstoßphase eine Solldruckkurve gemäß dem Franck-Starling-Gesetz zu erzeugen, wobei der Wert von $\Pi_i$ dem Wert des Drucks auf der Kurve über dem Zyklus $C_i$ entspricht.

5. Vorrichtung nach Anspruch 4, wobei die Solldruckkurve gemäß dem Franck-Starling-Gesetz für jede Ausstoßphase identisch ist.

6. Vorrichtung nach Anspruch 4, wobei die Solldruckkurve gemäß dem Franck-Starling-Gesetz in jeder Ausstoßphase neu definiert oder in regelmäßigen Zeitintervallen in Abhängigkeit von den Blutdruckwerten, die mit einer voreingestellten Frequenz an einer Körperextremität des Patienten gemessen werden, neu definiert wird.

7. Vorrichtung nach einem der Ansprüche 1 bis 3, wobei die Steuereinheit (40) konfiguriert ist, um bei jedem Zyklus $C_i$ den Solldruck $\Pi_i$ gemäß folgender Formel zu berechnen:

$$\Pi_i = (PM_s - K_s \cdot VE_{i-1} - R_s \cdot Q_{i-1})$$

Wobei:

- $PM_S$ der maximale systolische Druck ist, dessen Wert vorab parametriert und/oder eingestellt ist;
- $K_S$ ein Koeffizient zwischen 0,9 und 1,1 ist;
- $VE_{i-1}$ das Fluid-Volumen ist, das während der Ausstoßphase im vorherigen Zyklus $C_{i-1}$ in die Kammer (11) bewegt wurde;
- $R_S$ ein Koeffizient zwischen 0,4 und 0,6 ist;
- $Q_{i-1}$ der Fluiddurchsatz ist, der im vorherigen Zyklus $C_{i-1}$ aus der Kammer (11) ausgestoßen worden ist.

8. Vorrichtung nach einem der vorstehenden Ansprüche, wobei die Anzahl N an Zyklen $C_i$ zwischen 45 und 55 liegt und die Dauer $\Delta$t jedes Zyklus $C_i$ zwischen 0,004 s und 0,006 s liegt.

9. Vorrichtung nach einem der vorstehenden Ansprüche, wobei die Steuereinheit (40) konfiguriert ist, um die Ansaugphasen und die Ausstoßphasen des Fluids voneinander zu trennen, sodass sich während eines künstlichen Herzzyklus das in der Ansaugphase angesaugte Volumen von dem in den Ausstoßphasen ausgestoßenen Volumen unterscheidet.

10. Vorrichtung nach einem der vorstehenden Ansprüche, wobei:

- der erste Zyklus $C_1$ mit der natürlichen Systole zusammenfällt und zu dem Zeitpunkt beginnt, zu dem ein QRS-Komplex eines am Patienten (12) gemessenen EKG-Signals erkannt wird, oder
- sich der erste $C_1$-Zyklus im Verhältnis zur natürlichen Systole verzögert und nach einer Verzögerung zwischen 0,2 und 0,3 Sekunden ab dem Zeitpunkt beginnt, an dem ein QRS-Komplex eines am Patienten (12) gemessenen EKG-Elektrokardiogrammsignals erkannt wir

**Claims**

1. Device for the temporary extracorporeal circulatory assistance or replacement of the heart (13) of a patient (12), comprising:

- a linear actuator (10) configured to displace a member (70) in translation in a chamber (11) so as to cause a pulsatile fluid flow capable of supporting the activity of the heart of said patient, which flow is **characterised by** a succession of phases of aspiration and phases of ejection of the fluid,
- a control unit (40) configured to drive the actuator (10) and control the displacement of the member (70), which driving is carried out while taking into account input data originating from measurements of haemodynamic parameters of the patient (12), said unit displacing the member (70) over a distance D during each ejection phase,

**characterised in that** the control unit (40) is configured:

- to sequence the displacement through the distance D in an integer number N of cycles $C_i$ with N>1, each cycle having the same duration $\Delta$t,

- in each cycle $C_i$, to calculate a length $L_i$ of the displacement of the member (70) such that $D = \sum_{i=1}^{N} Li$, which length $L_i$ is calculated as a function of the input data derived from:

  ○ one or more haemodynamic parameter(s) of the patient (12), which are measured during the cycle $C_i$ or during one or more cycle(s) that precede(s) said cycle $C_i$ and are close to said cycle $C_i$,
  ○ the value of a setpoint pressure $\Pi_i$ based on the Frank-Starling law,

- in each cycle $C_i$, to transmit an instruction to the actuator (10) in order to displace the member (70) through the calculated length $L_i$.

2. Device according to claim 1, wherein the control unit (40) is configured to calculate the displacement length $L_i$ according to the following formula:

$$L_i = L_{i-1} + (\Pi_i \text{-} PR_i \text{-} \Delta P_{cor}) \times \frac{\Delta t^2}{S \times \mu}$$

where

- $L_{i-1}$ is the displacement length in the cycle $C_{i-1}$;
- $\Pi_i$ is the setpoint pressure in the cycle $C_i$;
- $PR_i$ is the actual pressure of the fluid as measured in the chamber (11) in the cycle $C_i$;
- $\Delta P_{cor}$ is a corrective pressure value,
- S is the equivalent cross section of the surface of the member (70) in contact with the fluid,
- $\mu$ is a constant.

3. Device according to claim 1, further comprising an ejection cannula (16) which can be implanted in the human body and is fluidically connected to the chamber (11), and wherein the control unit (40) is configured to calculate the displacement length $L_i$ according to the following formula:

$$L_i = L_{i-1} + (\Pi_i \text{-} PR_i) \times \frac{\Delta t^2}{S \times \mu}$$

where

- $L_{i-1}$ is the displacement length in the cycle $C_i$;
- $\Pi_i$ is the setpoint pressure in the cycle $C_i$;
- $PR_i$ is the actual pressure of the fluid as measured at the ejection cannula (16);
- S is the equivalent cross section of the surface of the member (70) in contact with the fluid,
- $\mu$ is a constant.

4. Device according to one of the preceding claims, wherein the control unit (40) is configured to generate a setpoint pressure curve according to the Frank-Starling law for each ejection phase, the value of $\Pi_i$ corresponding to the value of the pressure on said curve in the cycle $C_i$.

5. Device according to claim 4, wherein the setpoint pressure curve according to the Frank-Starling law is identical for each ejection phase.

6. Device according to claim 4, wherein the setpoint pressure curve according to the Frank-Starling law is redefined for each ejection phase or redefined at regular time intervals as a function of arterial pressure values measured with a preconfigured frequency, at one extremity of the body of the patient.

7. Device according to one of claims 1 to 3, wherein the control unit (40) is configured to calculate the setpoint pressure $\Pi_i$ for each cycle $C_i$ according to the following formula:

$$\Pi_i = (PM_s - K_s \cdot VE_{i-1} - R_s \cdot Q_{i-1})$$

where:

- $PM_S$ is the maximum systolic pressure whose value is preconfigured and/or adjusted;
- $K_S$ is a coefficient between 0.9 and 1.1;
- $VE_{i-1}$ is the volume of fluid set in movement in the chamber (11) during the ejection phase in the preceding cycle $C_{i-1}$;
- $R_S$ is a coefficient between 0.4 and 0.6;
- $Q_{i-1}$ is the fluid flow rate ejected from the chamber (11) in the preceding cycle $C_{i-1}$.

8. Device according to one of the preceding claims, wherein the number N of cycles $C_i$ is between 45 and 55 and the duration $\Delta t$ of each cycle $C_i$ is between 0.004 s and 0.006 s.

9. Device according to one of the preceding claims, wherein the control unit (40) is configured to dissociate the aspiration phases and the ejection phases of the fluid in such a way that, during an artificial cardiac cycle, the volume aspirated during the aspiration phase is different from the volume ejected during ejection phases.

**10.** Device according to one of the preceding claims, wherein:

- the first cycle $C_1$ coincides with the natural systole and begins at the instant when a QRS complex of an electrocardiography ECG signal measured on the patient (12) is detected, or
- the first cycle $C_1$ is offset with respect to the natural systole and begins after an offset of between 0.2 and 0.3 seconds from the instant when a QRS complex of an electrocardiography ECG signal measured on the patient (12) is detected.

[Fig. 1]

[Fig. 2]

[Fig. 3]

[Fig. 4]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2003032853 A
- WO 2006016047 A, BERTHIER
- US 2003032853 A1, KORAKIANITIS THEODOSIOS